# EUROPEAN PATENT SPECIFICATION

(11) **EP 0 421 379 B1**
(45) Date of publication and mention of the grant of the patent: **15.03.1995**
(21) Application number: 90118951.4
(22) Date of filing: 04.10.1990
(51) Int. Cl.: C07D 301/26, C07D 303/08

(54) **Preparation of monoepoxides**
Herstellung von Monoepoxiden
Préparation de monoépoxydes

(30) Priority: 04.10.1989 US 417126
(43) Date of publication of application: 10.04.1991
(73) Proprietor: THE DOW CHEMICAL COMPANY, Midland Michigan 48640-1967 (US)
(72) Inventor: Wernli, Walter L., Angleton, Texas 77515 (US); Shirtum, Robert P., Freeport, Texas 77541 (US)
(74) Representative: Sternagel, Hans-Günther, Dr.

(56) References cited:
- GB-A- 236 379
- GB-A- 1 015 033
- US-A- 1 446 872
- US-A- 3 061 615
- "Methoden der organischen Chemie", 4th edition, vol. VI/3, 1965, pages 374-375, Georg Thieme Verlag, Stuttgart, DE; HOUBEN-WEYL: "Sauerstoff-Verbindungen I"

## Description

The present invention concerns the preparation of monoepoxides from compounds containing only one vicinal halohydrin moiety per molecule.

Viriot et al. disclose in U S -A-No. 3,061,615 the preparation of epichlorohydrin by reacting a basic substance with an aqueous mixture of glycerol dichlorohydrin in the presence of a water-immiscible organic solvent for epichlorohydrin which circulates counter-current to the aqueous phase whereby the epichlorohydrin is separated from the organic solvent outside the dehydrochlorination apparatus and the organic solvent is continuously recycled to the dehydrochlorination apparatus for a fresh extraction of epichlorohydrin. The yield of epichlorohydrin is stated in the example to be 93.5 percent of theoretical based on dichlorohydrins.

In commercial processes, low yields result in large amounts of byproduct organic compounds which must be treated in bioponds so as to produce an environmentally acceptable effluent. It would therefore be desirable to have available a halohydrin process which produces monoepoxide compounds in high yields so as to reduce the load on bioponds. The present invention provides a method of producing monoepoxides, particularly epihalohydrins in yields of up to about 98% based on theoretical yield of halohydrin to monoepoxide.

The present invention pertains to a process for the preparation of compounds containing only one vicinal epoxide group per molecule which comprises
(1) continuously or intermittently contacting a mixture of (a) a compound having only one vicinal halohydrin moiety per molecule and (b) at least one organic solvent having a boiling point at least about 10°C above the boiling point of the monoepoxide being produced and which is essentially non-reactive with any of the compounds in the reaction mixture with (c) an aqueous solution of an alkali metal hydroxide;
(2) continuously removing, by codistilling or azeotroping, a mixture of water and resultant vicinal epoxide-containing compound from the reaction mixture; and
(3) separating the vicinal monoepoxide-containing compound from the water by any suitable means;
wherein said aqueous solution of an alkali metal hydroxide is added continuously or intermittently at a rate such that the monoepoxide compound being formed is removed essentially simultaneously with its formation.

The process of the present invention is conducted at a temperature which causes the mixture of monoepoxide and water to codistill or azeotrope from the reaction mixture at the pressure employed. Suitable such temperatures include, for example, from 10°C to 150°C, preferably from 20°C to 100°C, more preferably from 40°C to 80°C. At temperatures above 150°C, the reactants tend to boil out of the mixture before significant amounts of reaction can occur, and the reactants are afforded an opportunity to begin a self catalyzed reaction with each other to produce undesirable byproducts. At temperatures below 10°C the reaction kinetics are very slow and the caustic solutions could freeze solid and slow the reaction even more.

The reaction is usually conducted at a pressure which is sufficient to maintain the reactants in liquid form. Particularly suitable pressures include those from 1.3 kPa (10 mm Hg) absolute to 133 kPa (1000 mm Hg) absolute, preferably from 13.3 kPa (100 mm Hg) absolute to 53.2 kPa (400 mm Hg) absolute, more preferably from 19.9 kPa (150 mm Hg) absolute to 33.2 kPa (250 mm Hg) absolute. At pressures above 133 kPa (1000 mm Hg) absolute, temperatures for azeotropic or codistillation of the epoxide and water are sufficiently high so as to cause self catalyzed reaction of the reactants thereby producing undesirable byproducts. At pressures below 1.3 kPa (10 mm Hg) absolute, the reactants tend to boil out of solution before reaction to the desirable epoxide product can occur.

Any organic solvent which has a boiling point at least about 10°C , preferably at least about 20°C, more preferably at least about 45°C higher than the monoepoxide compound being produced can be employed herein. Particularly suitable organic solvents employed herein are the glycol ethers or mixtures thereof. Particularly suitable such glycol ethers include, for example, diethylene glycol dimethyl ether, diethylene glycol n-butyl ether, diethylene glycol ethyl ether, diethylene glycol methyl ether, dipropylene glycol methyl ether, ethylene glycol n-butyl ether, ethylene glycol ethyl ether, ethylene glycol methyl ether, ethylene glycol phenyl ether, propylene glycol phenyl ether, propylene glycol methyl ether, butylene glycol methyl ether, tripropylene glycol methyl ether or any combination thereof.

When the solvent has a boiling point close to that of the monoepoxide being produced, the product tends to self polymerize resulting in an undesirable polymeric or oligomeric byproduct. This is particularly true at the higher temperatures.

The solvent should not react appreciably with any of the compounds in the reaction mixture. Such reactions result in undesirable byproducts.

Suitable compounds containing only one vicinal halohydrin moiety per molecule which can be employed herein include, for example, 2-3 dichloro-1-propanol, 1,3-dichloro-2-propanol, 1,3-dibromo-2-propanol, 2,3-dibromo-1-propanol, ethylene chlorohydrin, 1-chloro-2-propanol, 1-chloro-2-butanol or any combination thereof.

Suitable alkali metal hydroxides which can be employed herein include, for example, sodium hydroxide, potassium hydroxide, lithium hydroxide or combinations thereof.

The alkali metal hydroxide is employed as an aqueous solution suitably at a concentration of from 1 to 80, preferably from 20 to 70, more preferably from 40 to 60 percent alkali metal hydroxide by weight.

The aqueous alkali metal hydroxide is added continuously or intermittently so as to provide essential removal of the monoepoxide compound being formed sufficiently simultaneously with its formation so as to reduce the formation of undesirable byproducts such as 1-chloro-2,3-dihydroxypropane, glycidol, diglycidyl ether and bis(chloromethyl)methoxy-2,3-epoxypropane. The alkali metal hydroxide is employed in an amount which provides suitably from 0.1 to 1.5, preferably from 0.9 to 1.1, more preferably from 0.98 to 1.05 moles of alkali metal hydroxide per vicinal halohydrin moiety.

The monoepoxide produced in the reaction is removed from the reaction mixture by continuously codistilling or azeotroping with water. The monoepoxide is then separated from the water by any suitable means such as liquid-liquid extraction, distillation, cold phase separations, carbon bed absorption or combinations thereof.

An alkali metal halide salt is formed as a byproduct during the reaction on a mole for mole basis with the monoepoxide. It can be removed from the reactor by any suitable means during the reaction, if desired, by removing liquid from the reactor and separating the salt therefrom by any suitable means and recycling the remaining contents to the reactor or else separating the compound containing only one vicinal halohydrin group per molecule therefrom and recycling it to the reactor. The salt can be separated by decantation, filtration, centrifugation, water washing or combinations thereof.

It is desired that the reactants be maintained in intimate contact during the reaction. One method for accomplishing this is by vigorous agitation during the reaction.

The rates of introduction of the reactant materials and the removal of products and byproducts is conducted such that the concentration of water in the liquid reaction medium is suitably less than about 4, preferably less than about 2, more preferably less than about 1.5 percent by weight based on the weight of total solution in the reactor.

In a preferred embodiment the reaction is conducted at a temperature of from 10°C to 150°C; a pressure of from 1.3 kPa (10 mm Hg) absolute to 133 kPa (1000 mm Hg) absolute; said organic solvent has a boiling point of at least about 10°C above the boiling point of the monoepoxide being produced; and byproduct are removed at a rate such that the water concentration in the liquid reaction medium is less than about 4 percent by weight based upon the weight of the liquid solution in the reactor; and wherein liquid is removed from the reactor, alkali metal halide salt is removed therefrom by any suitable means and the remaining contents returned to the reactor.

In a more preferred embodiment the reaction is conducted at a temperature of from 20°C to 100°C; a pressure of from 13.3 kPa (100 mm Hg) absolute to 53.2 kPa (400 mm Hg) absolute; said organic solvent has a boiling point of at least about 45°C above the boiling point of the monoepoxide being produced; and byproduct are removed at a rate such that the water concentration in the liquid reaction medium is less than about 2 percent by weight based upon the weight of the liquid solution in the reactor; and wherein liquid is removed from the reactor, alkali metal halide salt is removed therefrom by any suitable means and the remaining contents returned to the reactor.

In a most preferred embodiment the reaction is conducted at a temperature of from 40°C to 80°C; a pressure of from 19.9 kPa (150 mm Hg) absolute to 33.2 kPa (250 mm Hg) absolute; said organic solvent has a boiling point of at least about 45°C above the boiling point of the monoepoxide being produced; and byproduct are removed at a rate such that the water concentration in the liquid reaction medium is less than about 1.5 percent by weight based upon the weight of the liquid solution in the reactor; and wherein liquid is removed from the reactor, alkali metal halide salt is removed therefrom by any suitable means and the remaining contents returned to the reactor.

In a preferred embodiment said compound having only one vicinal halohydrin moiety per molecule is 2,3-dichloro-1-propanol; said alkali metal hydroxide is sodium hydroxide employed in a concentration of from 40 to 60 percent by weight in water; and said solvent is diethylene glycol dimethyl ether.

In another preferred embodiment, a mixture of 2,3-dichloro-1-propanol and organic solvent, preferably diethylene glycol dimethyl ether, is added to a continuously stirred vessel while an aqueous solution of alkali metal hydroxide, preferably sodium hydroxide as an aqueous solution in a concentration of from 15 to 50 percent by weight is continuously or intermittently added while simultaneously removing by codistillation or azeotropic distillation a mixture of water and epichlorohydrin and continuously or intermittently removing liquid reaction medium and subjecting it to filtration and recycle of reactants back to the reactor so as to prevent the buildup of byproduct sodium chloride in the reactor; and wherein the addition of reactants and removal of product and byproduct is adjusted such that the amount of water in the reactor is less than about 2 percent by weight based on the weight of liquid solution reactants (total solution in the reactor).

The following examples are for illustrative purposes only.

### EXAMPLE 1

### Preparation of epichlorohydrin (boiling point (b.p.)=115.2°C) from 2,3-dichloro-1-propanol in the presence of diethylene glycol dimethyl ether (b.p.=162°C).

A mixture composed of 800 grams of diglyme (diethylene glycol dimethyl ether) and 179.7 grams of 2,3-dichloro-1-propanol was heated to 70°C with vigorous agitation, and at reduced pressure (26.6 kPa (200 mm Hg)). To this heated, agitated mixture, a solution of 50 percent sodium hydroxide and water were pumped slowly (0.80 cm³/minute) into the heated, agitated mixture. A total of 111.44 grams (1 mole per vicinal chlorohydrin moiety) of 50 percent NaOH solution was used. The reaction of NaOH and 2,3-dichloro-1-propanol produced epichlorohydrin which azeotropically distilled with water at 67°C and 26.6 kPa (200 mm Hg) pressure. Salt (NaCl) formed and crystallized out in the solution of diglyme and 2,3-dichloro-1-propanol.

The epichlorohydrin/water azeotrope distillate was condensed and collected in a separate vessel and was allowed to separate into two phases. A total of 174.5 grams of distillate was collected.

The epichlorohydrin/water azeotrope distillate was removed at a rate such that the concentration of water in the reaction mixture was about 2 percent by weight.

The two phases from the distillate and the reactor solution were analyzed for epichorohydrin content and impurities concentrations. Of the theoretical 128.85 grams of epichlorohydrin which could be produced by the reaction, 127.3 grams could be accounted for as epichlorohydrin. Therefore, the reaction yields to epichlorohydrin were at least 98 percent based on 2,3-dichloro-1-propanol.

### COMPARATIVE EXPERIMENT A

### Preparation of epichlorohydrin (b.p.=115.2°C) from 2,3-dichloro-1-propanol in the presence of 1,2,3-trichloropropane (b.p.=156.17°C, but reacts with starting material).

A mixture composed of 1432 grams of 1,2,3-trichloropropane, 95 grams of diglyme (diethylene glycol dimethyl ether) and 166.7 grams of 2,3-dichloro-1-propanol was heated to 70°C with vigorous agitation, and at reduced pressure (26.6 kPa (200 mm Hg)). To this heated, agitated mixture, a solution of 50 percent sodium hydroxide and water were pumped slowly (0.80 cm³/minute) into the heated, agitated mixture. A total of 119.0 grams (1.151 moles per vicinal chlorohydrin moiety) of 50 percent NaOH solution was used. The reaction of NaOH and 2,3-dichloro-1-propanol produced epichlorohydrin which azeotropically distilled with water at 67°C and 26.6 kPa (200 mm Hg) pressure. Salt (NaCl) formed and crystallized out in the solution of diglyme, 1,2,3,trichloropropane and 2,3-dichloro-1-propanol.

The epichlorohydrin/water azeotrope distillate was condensed and collected in a separate vessel and was allowed to separate into two phases. A total of 89.9 grams of distillate was collected.

The epichlorohydrin/water azeotrope distillate was removed at a rate such that the concentration of water in the reaction mixture was about 3.6 percent by weight.

The two phases from the distillate and the reactor solution were analyzed for epichlorohydrin content and impurities concentrations. Of the theoretical 119.5 grams of epichlorohydrin which could be produced by the reaction, 23.9 grams could be accounted for as epichlorohydrin. Therefore, the reaction conversion to epichorohydrin was 20 percent which is unacceptable in a commercial process. Analysis revealed that the major portion of the NaOH was consumed converting 1,2,3-trichloropropane into 1,3-dichloropropene instead of epoxidizing 2,3-dichloropropanol to epichlorohydrin.

### EXAMPLE 2

### Preparation of epichlorohydrin from 2,3-dichloro-1-propanol in presence of diethylene glycol dimethyl ether (b.p.=162°C).

A mixture composed of 709 grams of diglyme (diethylene glycol dimethyl ether) and 141.9 grams of 2,3-dichloro-1-propanol was heated to 70°C with vigorous agitation, and at reduced pressure (26.6 kPa (200 mm Hg)). To this heated, agitated mixture, a solution of 50 percent sodium hydroxide and water were pumped slowly (0.50 cm³/minute) into the heated, agitated mixture. A total of 87.9 grams (1 mole per vicinal chlorohydrin moiety) of 50 percent NaOH solution was used. The reaction of NaOH and 2,3-dichloro-1-propanol produced epichlorohydrin which azeotropically distilled with water at 67°C and 26.6 kPa (200 mm Hg) pressure. Salt (NaCl) formed and crystallized out in the solution of diglyme and 2,3-dichloro-1-propanol.

The epichlorohydrin/water azeotrope distillate was condensed and collected in a separate vessel and was allowed to separate into two phases. A total of 167.7 grams of distillate was collected.

The epichlorohydrin/water azeotrope distillate was removed at a rate such that the concentration of water in the reaction mixture was about 1.5 percent by weight

The two phases from the distillate and the reactor solution were analyzed for epichlorohydrin content and impurities concentrations. Of the theoretical 101.55 grams of epichlorohydrin which could be produced by the reaction, 98.8 grams could be accounted for as epichlorohydrin. Therefore, the reaction yields to epichlorohydrin were at least 97 percent.

## Claims

1. A process for the preparation of compounds containing only one vicinal epoxide group per molecule which comprises
(1) continuously or intermittently contacting a mixture of (a) a compound having only one vicinal halohydrin moiety per molecule and (b) at least one organic solvent having a boiling point at least about 10°C above the boiling point of the monoepoxide being produced and which is essentially non-reactive with any of the compounds in the reaction mixture with (c) an aqueous solution of an alkali metal hydroxide;
(2) continuously removing, by codistilling or azeotroping, a mixture of water and resultant vicinal epoxide-containing compound from the reaction mixture; and
(3) separating the vicinal monoepoxide-containing compound from the water by any suitable means;
wherein said aqueous solution of an alkali metal hydroxide is added continuously or intermittently at a rate such that the monoepoxide compound being formed is removed essentially simultaneously with its formation.

2. A process of Claim 1 wherein the reaction is conducted at a temperature of from 10°C to 150°C; a pressure of from 1.3 kPa (10 mm Hg) absolute to 133 kPa (1000 mm Hg) absolute; said organic solvent has a boiling point of at least about 10°C above the boiling point of the monoepoxide being produced; and byproduct are removed at a rate such that the water concentration in the liquid reaction medium is less than about 4 percent by weight based upon the weight of the liquid solution in the reactor; and wherein liquid is removed from the reactor, alkali metal halide salt is removed therefrom by any suitable means and the remaining contents returned to the reactor.

3. A process of Claim 1 wherein the reaction is conducted at a temperature of from 20°C to 100°C; a pressure of from 13.3 kPa (100 mm Hg) absolute to 53.2 kPa (400 mm Hg) absolute; said organic solvent has a boiling point of at least about 45°C above the boiling point of the monoepoxide being produced; and byproduct are removed at a rate such that the water concentration in the liquid reaction medium is less than about 2 percent by weight based upon the weight of the liquid solution in the reactor; and wherein liquid is removed from the reactor, alkali metal halide salt is removed therefrom by any suitable means and the remaining contents returned to the reactor.

4. A process of Claim 1 wherein the reaction is conducted at a temperature of from 40°C to 80°C; a pressure of from 19.9 kPa (150 mm Hg) absolute to 33.2 kPa (250 mm Hg) absolute; said organic solvent has a boiling point of at least about 45°C above the boiling point of the monoepoxide being produced; and byproduct are removed at a rate such that the water concentration in the liquid reaction medium is less than about 1.5 percent by weight based upon the weight of the liquid solution in the reactor; and wherein liquid is removed from the reactor, alkali metal halide salt is removed therefrom by any suitable means and the remaining contents returned to the reactor.

5. A process of Claim 1, 2, 3 or 4 wherein said compound having only one vicinal halohydrin moiety per molecule is 2-3 dichloro-1-propanol, 1,3-dichloropropanol, 1,3-dibromo-2-propanol, 2,3-dibromo-1-propanol, ethylene chlorohydrin, propylene chlorohydrin, butylene chlorohydrin, or any combination thereof; said alkali metal hydroxide is sodium hydroxide or potassium hydroxide employed in a concentration of from 1 to 80 percent by weight in water; and said solvent is a glycol ether.

6. A process of Claim 1, 2, 3 or 4 wherein said compound having only one vicinal halohydrin moiety per molecule is 2-3 dichloro-1-propanol, 1,3-dichloropropanol, 1,3-dibromo-2-propanol, 2,3-dibromo-1-propanol, or any combination thereof; said alkali metal hydroxide is sodium hydroxide or potassium hydroxide employed in a concentration of from 20 to 70 percent by weight in water; and said solvent is diethylene glycol dimethyl ether, diethylene glycol methyl ether, dipropylene glycol methyl ether, ethylene glycol n-butyl ether, ethylene glycol ethyl ether, ethylene glycol methyl ether, propylene glycol methyl ether, butylene glycol methyl ether, or any combination thereof.

7. A process of Claim 1, 2, 3 or 4 wherein said compound having only one vicinal halohydrin moiety per molecule is 2,3-dichloro-1-propanol; said alkali metal hydroxide is sodium hydroxide employed in a concentration of from 40 to 60 percent by weight in water; and said solvent is diethylene glycol dimethyl ether.

## Patentansprüche

1. Verfahren zur Herstellung von Verbindungen, die nur eine vicinale Epoxidgruppe pro Molekül aufweisen, durch:
(1) kontinuierliches oder absatzweises Inberührungbringen einer Mischung von (a) einer Verbindung mit nur einer vicinalen Halohydringruppe pro Molekül und (b) mindestens einem organischen Lösemittel mit einem Siedepunkt von mindestens etwa 10° C über dem Siedepunkt des sich bildenden Monoepoxids und das im wesentlichen inert gegenüber allen Verbindungen in der Reaktionsmischung ist, mit (c) einer wäßrigen Lösung eines Alkalihydroxids,
(2) kontinuierliches Entfernen durch Codestillation oder azeotrope Destillation einer Mischung von Wasser und entstehender, vicinales Epoxid enthaltender Verbindung aus der Reaktionsmischung und
(3) Abtrennen der vicinale Monoepoxidgruppe enthaltenden Verbindung vom Wasser durch jede geeignete Maßnahme, wobei die wäßrige Alkalihydroxidlösung kontinuierlich oder absatzweise mit einer solchen Geschwindigkeit zugesetzt wird, daß die gebildete Monoepoxidverbindung im wesentlichen gleichzeitig mit ihrer Bildung entfernt wird.

2. Verfahren nach Anspruch 1,
**dadurch gekennzeichnet**,
daß die Reaktion bei einer Temperatur von 10°C bis 150°C, einem Druck von 1,3 kPa (10 mm Hg) absolut bis 133 kPa (1000 mm Hg) absolut ausgeführt wird, das organische Lösemittel einen Siedepunkt aufweist, der mindestens etwa 10° C über dem Siedepunkt des sich bildenden Monoepoxids liegt, und das Nebenprodukt mit einer solchen Geschwindigkeit entfernt wird, daß die Wasserkonzentration im flüssigen Reaktionsmedium weniger als etwa 4 Gew.-%, bezogen auf Gewicht der flüssigen Lösung im Reaktor, beträgt und wobei Flüssigkeit aus dem Reaktor entfernt wird, Alkalihalogenidsalz durch jede geeignete Maßnahme daraus entfernt wird und der verbleibende Inhalt in den Reaktor zurückgeführt wird.

3. Verfahren nach Anspruch 1,
**dadurch gekennzeichnet**,
daß die Reaktion bei einer Temperatur von 20°C bis 100°C, einem Druck von 13,3 kPa (100 mm Hg) absolut bis 53,2 kPa (400 mm Hg) absolut ausgeführt wird, das organische Lösemittel einen Siedepunkt aufweist, der mindestens etwa 45°C über dem Siedepunkt des sich bildenden Monoepoxids liegt, und das Nebenprodukt mit einer solchen Geschwindigkeit entfernt wird, daß die Wasserkonzentration im flüssigen Reaktionsmedium weniger als etwa 2 Gew.-%, bezogen auf Gewicht der flüssigen Lösung im Reaktor, beträgt und wobei Flüssigkeit aus dem Reaktor entfernt wird, Alkalihalogenidsalz durch jede geeignete Maßnahme daraus entfernt wird und der verbleibende Inhalt in den Reaktor zurückgeführt wird.

4. Verfahren nach Anspruch 1,
**dadurch gekennzeichnet**,
daß die Reaktion bei einer Temperatur von 40°C bis 80°C, einem Druck von 19,9 kPa (150 mm Hg) absolut bis 33,2 kPa (250 mm Hg) absolut ausgeführt wird, das organische Lösemittel einen Siedepunkt aufweist, der mindestens etwa 45°C über dem Siedepunkt des sich bildenden Monoepoxids liegt, und das Nebenprodukt mit einer solchen Geschwindigkeit entfernt wird, daß die Wasserkonzentration im flüssigen Reaktionsmedium weniger als etwa 1,5 Gew.-%, bezogen auf Gewicht der flüssigen Lösung im Reaktor, beträgt und wobei Flüssigkeit aus dem Reaktor entfernt wird, Alkalihalogenidsalz durch jede geeignete Maßnahme daraus entfernt wird und der verbleibende Inhalt in den Reaktor zurückgeführt wird.

5. Verfahren nach Anspruch 1, 2, 3 oder 4,
**dadurch gekennzeichnet**,
daß die Verbindung mit nur einer vicinalen Halohydringruppe pro Molekül 2,3-Dichlor-1-propanol, 1,3-Dichlorpropanol, 1,3-Dibrom-2-propanol, 2,3-Dibrom-1-propanol, Ethylenchlorhydrin, Propylenchlorhydrin, Butylenchlorhydrin oder eine Kombination derselben ist, das Alkalihydroxid Natriumhydroxid oder Kaliumhydroxid ist und in einer Konzentration von 1 bis 80 Gew.-% in Wasser verwendet wird und das Lösemittel ein Glycolether ist.

6. Verfahren nach Anspruch 1, 2, 3 oder 4,
**dadurch gekennzeichnet**,
daß die Verbindung mit nur einer vicinalen Halohydringruppe pro Molekül 2,3-Dichlor-1-propanol, 1,3-Dichlorpropanol, 1,3-Dibrom-2-propanol, 2,3-Dibrom-1-propanol, oder eine Kombination derselben ist, das Alkalihydroxid Natriumhydroxid oder Kaliumhydroxid ist und in einer Konzentration von 20 bis 70 Gew.-% in Wasser verwendet wird und das Lösemittel Diethylenglycoldimethylether, Diethylenglycolmethylether, Dipropylenglycolmethylether, Ethylenglycol-n-butylether, Ethylenglycolethylether, Ethylenglycolmethylether, Propylenglycolmethylether, Butylenglycolmethylether oder eine Kombination derselben ist.

7. Verfahren nach Anspruch 1, 2, 3 oder 4,
**dadurch gekennzeichnet**,
daß die Verbindung mit nur einer vicinalen Halohydringruppe pro Molekül 2,3-Dichlor-1-propanol ist, das Alkalihydroxid Natriumhydroxid ist, verwendet in einer Konzentration von 40 bis 60 Gew.-% in Wasser, und das Lösemittel Diethylenglycoldimethylether ist.

## Revendications

1. Procédé de préparation de composés contenant seulement un groupe époxy vicinal par molécule, procédé qui comprend les étapes consistant à
(1) mettre en contact, de manière continue ou intermittente, un mélange de (a) un composé présentant seulement un fragment halogénohydrine vicinal par molécule et (b) au moins un solvant organique ayant un point d'ébullition supérieur d'au moins environ 10°C au point d'ébullition du monoépoxyde produit et pratiquement inerte vis-à-vis de l'un quelconque des composés du mélange réactionnel, avec (c) une solution aqueuse d'un hydroxyde de métal alcalin,
(2) éliminer de manière continue du mélange réactionnel, par codistillation ou distillation azéotropique, un mélange d'eau et du composé époxyde vicinal résultant, et
(3) séparer le composé monoépoxyde vicinal de l'eau par tout moyen approprié,
procédé dans lequel ladite solution aqueuse d'hydroxyde de métal alcalin est ajoutée, de manière continue ou intermittente, à une vitesse telle que le composé monoépoxyde formé est éliminé pratiquement en même temps qu'il se forme.

2. Procédé selon la revendication 1, dans lequel la réaction est réalisée à une température de 10°C à 150°C, sous une pression absolue de 1,3 kPa (10 mm de mercure) à 133 kPa (1000 mm de mercure), ledit solvant organique a un point d'ébullition supérieur d'au moins environ 10°C au point d'ébullition du monoépoxyde produit, et les sous-produits sont éliminés à une vitesse telle que la concentration de l'eau dans le milieu réactionnel liquide est inférieure à environ 4% en poids, par rapport au poids de la solution liquide dans le réacteur, et dans lequel du liquide est éliminé du réacteur, l'halogénure de métal alcalin est éliminé dudit liquide par tout moyen approprié et ce qui reste est renvoyé dans le réacteur.

3. Procédé selon la revendication 1, dans lequel la réaction est réalisée à une température de 20°C à 100°C, sous une pression absolue de 13,3 kPa (100 mm de mercure) à 53,2 kPa (400 mm de mercure), ledit solvant organique a un point d'ébullition supérieur d'au moins environ 45°C au point d'ébullition du monoépoxyde produit, et les sous-produits sont éliminés à une vitesse telle que la concentration de l'eau dans le milieu réactionnel liquide est inférieure à environ 2% en poids, par rapport au poids de la solution liquide dans le réacteur, et dans lequel du liquide est éliminé du réacteur, l'halogénure de métal alcalin est éliminé dudit liquide par tout moyen approprié et ce qui reste est renvoyé dans le réacteur.

4. Procédé selon la revendication 1, dans lequel la réaction est réalisée à une température de 40°C à 80°C, sous une pression absolue de 19,9 kPa (150 mm de mercure) à 33,2 kPa (250 mm de mercure), ledit solvant organique a un point d'ébullition supérieur d'au moins environ 45°C au point d'ébullition du monoépoxyde produit, et les sous-produits sont éliminés à une vitesse telle que la concentration de l'eau dans le milieu réactionnel liquide est inférieure à environ 1,5% en poids, par rapport au poids de la solution liquide dans le réacteur, et dans lequel du liquide est éliminé du réacteur, l'halogénure de métal alcalin est éliminé dudit liquide par tout moyen approprié et ce qui reste est renvoyé dans le réacteur.

5. Procédé selon l'une quelconque des revendications 1 à 4, dans lequel ledit composé présentant seulement un fragment halogénohydrine vicinal par molécule est le 2,3-dichloro-1-propanol, le 1,3-dichloropropanol, le 1,3-dibromo-2-propanol, le 2,3-dibromo-1-propanol, l'éthylènechlorhydrine, la propylènechlorhydrine, la butylènechlorhydrine ou une combinaison quelconque de ces composés, ledit hydroxyde de métal alcalin est l'hydroxyde de sodium ou l'hydroxyde de potassium, qui est employé à une concentration de 1 à 80% en poids dans de l'eau, et ledit solvant est un éther de glycol.

6. Procédé selon l'une quelconque des revendications 1 à 4, dans lequel ledit composé présentant seulement un fragment halogénohydrine vicinal par molécule est le 2,3-dichloro-1-propanol, le 1,3-dichloropropanol, le 1,3-dibromo-2-propanol, le 2,3-dibromo-1-propanol ou une combinaison quelconque de ces composés, ledit hydroxyde de métal alcalin est l'hydroxyde de sodium ou l'hydroxyde de potassium, qui est employé à une concentration de 20 à 70% en poids dans de l'eau, et ledit solvant est l'éther diméthylique du diéthylèneglycol, l'éther méthylique du diéthylèneglycol, l'éther méthylique du dipropylèneglycol, l'éther n-butylique de l'éthylèneglycol, l'éther éthylique de l'éthylèneglycol, l'éther méthylique de l'éthylèneglycol, l'éther méthylique du propylèneglycol, l'éther méthylique du butylèneglycol ou une combinaison quelconque de ces composés.

7. Procédé selon l'une quelconque des revendications 1 à 4, dans lequel ledit composé présentant seulement un fragment halogéno hydrine vicinal par molécule est le 2,3-dichloro-1-propanol, ledit hydroxyde de métal alcalin est l'hydroxyde de sodium qui est employé à une concentration de 40 à 60% en poids dans de l'eau, et ledit solvant est l'éther diméthylique du diéthylèneglycol.
